# EUROPEAN PATENT APPLICATION

(11) **EP 4 741 510 A1**
(43) Date of publication of application: **13.05.2026**
(21) Application number: 24863179.8
(22) Date of filing: 04.09.2024
(51) Int. Cl.: C12P 13/10, B01D 15/36

(54) **METHOD FOR PRODUCING HIGH-PURITY ARGININE**

(30) Priority: 05.09.2023 KR 20230117455
(71) Applicant: CJ Cheiljedang Corporation, Seoul 04560 (KR)
(72) Inventor: HAN, Hyeong Seok, Seoul 04560 (KR); KIM, Joongho, Seoul 04560 (KR)
(74) Representative: Meissner Bolte Partnerschaft mbB
(86) International application number: PCT/KR2024/013309
(87) International publication number: WO 2025/053599

(57) **Abstract**

The present application relates to a method for producing arginine, wherein a bleaching step is not performed, and an anion exchange step is separated and performed simultaneously with an arginine production step, thereby improving the yield of arginine and significantly reducing the time required for production.

## Description

### [TECHNICAL FIELD]

### CROSS-REFERENCE TO RELATED APPLICATION(S)

This application claims the benefit of priority based on Korean Patent Application No. 10-2023-0117455 filed on September 5, 2023, and all contents disclosed in the document of the corresponding Korean Patent Application are incorporated herein by reference.

The present application relates to a method for producing high-purity arginine.

### [BACKGROUND ART]

The present invention relates to a method for producing high-purity arginine used in animal feed additives, etc., from arginine fermentation broth. The conventional method for producing arginine includes the steps of separating bacterial cells from the arginine fermentation broth, cation exchange process, decolorization process, anion exchange process, followed by crystallization and separation. Among these, the decolorization process is a step in which activated carbon is added to absorb and separate organic impurities or colored ionic substances contained in the arginine process liquid, thereby improving the purity of the arginine solution and the transmittance of arginine. However, a demand for high transmittance in arginine products used for animal feed additives has been decreasing recently, and environmental issues concerning the disposal of used activated carbon and wastewater from the decolorization process are becoming increasingly prominent.

### [PRIOR ART]

(Patent document 1) US 2003-0124686 A1

### [DISCLOSURE]

### [TECHNICAL PROBLEM]

An object of the present application is to provide a method for producing arginine.

### [TECHNICAL SOLUTION]

The present application relates to a method for producing arginine in which a decolorization process is not performed, and an anion exchange process is separated and carried out concurrently with the arginine production process, thereby the yield of arginine is enhanced and the time required for arginine production is drastically reduced.

An aspect provides a method for producing arginine.

The arginine may be arginine crystals or arginine powder.

### Definition of Terms

In the present application, the term "fermentation product" may mean a resultant product of enzymatic or metabolic decomposition of organic material utilizing a microorganism. For example, the fermentation product may include a culture product itself obtained by culturing the microorganism in a culture medium, the culture product from which cells (the microorganism, or a part of the microorganism) are removed, or a concentrated product, a dry product, or a lyophilized product of the culture product from which the bacterial cells are removed. Furthermore, in this case, the term "fermentation broth" may include the whole fermentation product containing arginine produced from the microorganism, or may be one from which impurities are removed from the fermentation product.

In the present application, the term "eluate" may mean the liquid having passed through a cation exchange resin and/or an anion exchange resin.

In the present application, the term "process liquid" may refer collectively to any liquid that is introduced into each step for producing arginine (e.g., arginine crystals) and/or any liquid obtained after each processing step. For example, the process liquid may include a fermentation broth, eluate, concentrate, filtrate containing arginine, and the like.

In this application, the term "mother liquor" may refer to the liquid remaining after separation of arginine (e.g., arginine crystals) by performing a concentration-crystallization process on a process liquid containing arginine

### Fermentation broth containing arginine

An aspect may provide a method for producing arginine crystals from the fermentation broth containing arginine.

The fermentation broth containing arginine may be obtained by culturing a microorganism producing arginine. The microorganism producing arginine includes both wild-type microorganisms or microorganisms having natural or artificial genetic modification. Such a microorganism may contain a genetic modification of a target protein for arginine production, and may be a microorganism in which a specific mechanism is weakened or enhanced due to any cause such as insertion of a foreign gene, or strengthened or inactivated activity of intrinsic gene.

The microorganism producing arginine according to the present application may be a microorganism naturally possessing arginine production capability, or a microorganism on which arginine production capability is conferred to a microorganism lacking arginine production capability, but is not limited thereto. Specifically, the microorganism producing arginine or the microorganism having arginine production capability in the present application, may be a microorganism in which a part of the genes within the arginine biosynthetic pathway is enhanced or weakened, or a part of the genes within the arginine degradation pathway is enhanced or weakened. In the present application, the terms "enhanced" or "increased" arginine production capability of the microorganism mean that the arginine production capability of the microorganism of the present application is improved, compared with other microorganisms, parental strains (or mother strains), or non-modified microorganisms other than the microorganism of the present application. For example, the arginine production capability of the microorganism in the present application may be improved by about 1% or more, 2% or more, 5% or more, 10% or more, 100% or more, 200% or more, 500% or more, 1000% or more, 1100% or more, 1200% or more, or 1300% or more compared with the arginine production capability of other microorganisms, but is not limited thereto. The term "about" includes a range including ±0.5, ±0.4, ±0.3, ±0.2, ±0.1, etc., and includes all values within a range equivalent or similar to the numerical value following the term about, but is not limited thereto.

The microorganism producing arginine may be a microorganism of *Escherichia sp.,* a microorganism of *Bacillus sp.,* a microorganism of *Saccharomyces sp.,* or *Corynebacterium sp.* In the case where the microorganism is a microorganism of *Escherichia sp.,* the microorganism may be *Escherichia coli.* In the case where the microorganism is a microorganism of *Bacillus sp.,* the microorganism may be one or more selected from the group consisting of *Bacillus subtilis, Bacillus vulgaris,* and *Bacillus cereus,* and the like. In the case where the microorganism is a microorganism of *Saccharomyces sp.,* the microorganism may be *Saccharomyces cerevisiae.* In the case that the microorganism is a microorganism of *Corynebacterium sp.,* the microorganism may be one or more selected from the group consisting of *Corynebacterium glutamicum, Corynebacterium erythrogenes, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris, Corynebacterium crenatum,* and *Corynebacterium flavescens,* and for example, it may be *Corynebacterium glutamicum,* but is not limited thereto.

The culturing of the microorganism producing arginine may be performed according to suitable media and culture conditions known in the art. This culturing process can be easily adjusted and used by those skilled in the art depending on the selected strain. Specifically, the culturing may be batch, continuous, and fed-batch, but is not limited thereto. In the present application, the term, "medium," means substances mixed mainly with nutrients required for culturing the microorganism, and supplys nutrients and growth factors as well as water indispensable for survival and growth. Specifically, the medium and other culture conditions used for culturing the microorganism of the present application can be any medium used for culturing generally microorganisms without particular limitation. However, the microorganism of the present application may be cultured while controlling temperature, pH, and the like, under aerobic conditions in a general medium containing suitable carbon sources, nitrogen sources, phosphorus sources, inorganic compounds, amino acids and/or vitamins, and the like.

In one example, the fermentation broth containing arginine may be one from which cells (e.g., the microorganism producing arginine, or a part of the microorganism, etc.) are removed.

Removing the cells from the fermentation broth may be performed by conventional methods such as separation (e.g., centrifugation, etc.), filtration (e.g., use of cell separation equipment, ceramic membrane filters, and the like)

### Step (a): A step of passing the fermentation broth containing arginine through a cation exchange resin (cation exchange process)

The method for producing arginine of the present application may comprise a step of passing the fermentation broth containing arginine through a cation exchange resin.

The cation exchange resin may remove the cationic impurities. The cation exchange resin may be a conventionally known cation exchange resin, for example, strongly acidic cation exchange resin (SAC) or weakly acidic cation exchange resin (WAC), but is not limited thereto. The cation exchange resin may be a gel type or a porous type, but is not limited thereto.

The cationic impurities may be alkali metal ions (e.g., sodium ion, potassium ion, etc.), alkaline earth metal ions (e.g., magnesium ion, calcium ion, etc.), or ammonium ion, and the like.

The cation exchange process may comprise a step of treating an eluent in order to desorb arginine adsorbed to the cation exchange resin.

The eluent may be a substance conventionally used to separate substances remaining on a stationary phase without limitation, and in one example, sodium hydroxide or aqueous ammonia solution may be used as the eluent, but is not limited thereto.

In one example, the eluent may be used in diluted for at various concentrations, and for example, it may be used at a concentration of 0.01 to 5 N, 0.01 to 4 N, 0.01 to 3 N, 0.01 to 2 N, 0.01 to 1 N, 0.05 to 5 N, 0.05 to 4 N, 0.05 to 3 N, 0.05 to 2 N, 0.05 to 1 N, 0.1 to 5 N, 0.1 to 4 N, 0.1 to 3 N, 0.1 to 2 N, 0.1 to 1 N, 0.5 to 5 N, 0.5 to 4 N, 0.5 to 3 N, 0.5 to 2 N, 0.5 to 1 N, 1 to 5 N, 1 to 4 N, 1 to 3 N, 1 to 2 N, 2 to 5 N, 2 to 4 N, 2 to 3 N, 2.5 to 5 N, 2.5 to 4 N, or 2.5 to 3 N.

The cation exchange process may comprise a step of concentrating the eluate under a high temperature condition in order to remove the used eluent from the eluate obtained in the cation exchange process after the step of treating the eluent.

The high temperature condition may be set without limitation as long as the condition can remove the used eluent (e.g., aqueous ammonia solution, etc.), and in one example, it may be 30 °C or higher, 35 °C or higher, 40 °C or higher, 45 °C or higher, 50 °C or higher, 55 °C or higher, or 60 °C or higher.

The step of concentrating may be a step of concentrating the eluate to a concentration prior to the point at which arginine is obtained as crystals.

The step of concentrating may be a step of concentrating the eluate until the concentration of arginine in the concentrate reaches 100 g/l or less, 110 g/l or less, 120 g/l or less, 130 g/l or less, 140 g/l or less, 150 g/l or less, 160 g/l or less, 170 g/l or less, 180 g/l or less, 190 g/l or less, 200 g/l or less, 210 g/l or less, 220 g/l or less, 230 g/l or less, 240 g/l or less, 250 g/l or less, 30 to 100 g/l, 30 to 110 g/l, 30 to 120 g/l, 30 to 130 g/l, 30 to 140 g/l, 30 to 150 g/l, 30 to 160 g/l, 30 to 170 g/l, 30 to 180 g/l, 30 to 190 g/l, 30 to 200 g/l, 30 to 210 g/l, 30 to 220 g/l, 30 to 230 g/l, 30 to 240 g/l, 30 to 250 g/l, 40 to 100 g/l, 40 to 110 g/l, 40 to 120 g/l, 40 to 130 g/l, 40 to 140 g/l, 40 to 150 g/l, 40 to 160 g/l, 40 to 170 g/l, 40 to 180 g/l, 40 to 190 g/l, 40 to 200 g/l, 40 to 210 g/l, 40 to 220 g/l, 40 to 230 g/l, 40 to 240 g/l, 40 to 250 g/l, 50 to 100 g/l, 50 to 110 g/l, 50 to 120 g/l, 50 to 130 g/l, 50 to 140 g/l, 50 to 150 g/l, 50 to 160 g/l, 50 to 170 g/l, 50 to 180 g/l, 50 to 190 g/l, 50 to 200 g/l, 50 to 210 g/l, 50 to 220 g/l, 50 to 230 g/l, 50 to 240 g/l, 50 to 250 g/l, 60 to 100 g/l, 60 to 110 g/l, 60 to 120 g/l, 60 to 130 g/l, 60 to 140 g/l, 60 to 150 g/l, 60 to 160 g/l, 60 to 170 g/l, 60 to 180 g/l, 60 to 190 g/l, 60 to 200 g/l, 60 to 210 g/l, 60 to 220 g/l, 60 to 230 g/l, 60 to 240 g/l, 60 to 250 g/l, 70 to 100 g/l, 70 to 110 g/l, 70 to 120 g/l, 70 to 130 g/l, It may be a step of concentrating to 70 to 140 g/l, 70 to 150 g/l, 70 to 160 g/l, 70 to 170 g/l, 70 to 180 g/l, 70 to 190 g/l, 70 to 200 g/l, 70 to 210 g/l, 70 to 220 g/l, 70 to 230 g/l, 70 to 240 g/l, or 70 to 250 g/l, but is not limited thereto.

### Step (b): A step of concentrating and crystallizing the process liquid obtained in step (a) (concentration-crystallization process)

The method for producing arginine of the present application may comprise a step of concentrating and crystallizing (concentration-crystallization) the process liquid obtained in step (a).

The process liquid obtained in step (a) may be an eluate obtained by passing a fermentation broth containing arginine through a cation exchange process, or a concentrate obtained by subjecting the eluate to a concentration process.

The concentration-crystallization process may be a step of concentrating arginine to a concentration at which it is obtained as a crystal. The concentration and crystallization process is performed so that the concentration of arginine in the concentrate is 200 g/l or more, 250 g/l or more, 300 g/l or more, 350 g/l or more, 400 g/l or more, 450 g/l or more, 500 g/l or more, 550 g/l or more, 600 g/l or more, 650 g/l or more (the upper limit is about 700 g/l), 200 to 700 g/l, 200 to 650 g/l, 200 to 600 g/l, 200 to 550 g/l, 200 to 500 g/l, 250 to 700 g/l, 250 to 650 g/l, 250 to 600 g/l, 250 to 550 g/l, 250 to 500 g/l, 300 to 700 g/l, 300 to 650 g/l, 300 to 600 g/l, 300 to 550 g/l, 300 to 500 g/l, 350 to 700 g/l, 350 to 650 g/l, 350 to 600 g/l, 350 to 550 g/l, 350 to 500 g/l, 400 to 700 g/l, 400 to 650 g/l, 400 to 600 g/l, 400 to 550 g/l, 400 to 500 g/l, 450 to 700 g/l, 450 to 650 g/l, 450 to 600 g/l, 450 to 550 g/l, 450 to 500 g/l, 500 to 700 g/l, 500 to 650 g/l, 500 to 600 g/l, or 500 to 550 g/l, but is not limited to.

The concentration-crystallization process can be performed under temperature conditions that can obtain arginine as crystals. In one example, the concentration-crystallization process is performed at a temperature of 30 to 80°C, 30 to 75°C, 30 to 70°C, 30 to 65°C, 30 to 60°C, 30 to 55°C, 30 to 50°C, 35 to 80°C, 35 to 75°C, 35 to 70°C, 35 to 65°C, 35 to 60°C, 35 to 55°C, 35 to 50°C, 40 to 80°C, 40 to 75°C, 40 to 70°C, 40 to 65°C, 40 to 60°C, 40 to 55°C, 40 to 50°C, 45 to 80°C, 45 to 75°C, 45 to 70°C, 45 to 65°C, 45 to 60°C, 45 to 55°C, 45 to 50°C, 50 to 80°C, 50 to 75°C, 50 to 70°C, 50 to 65°C, 50 to 60°C, or 50 to 55°C, for example 55°C.

The concentration-crystallization process may further comprise a cooling step to reduce the solubility of arginine and increase the yield of arginine crystals. The cooling step is to cool the arginine concentrate to 40 °C or less, 35 °C or less, 30 °C or less, 25 °C or less, 20 °C or less, 15 °C or less, 10 °C or less, 5 °C or less (the lower limit is 0 °C), 0 to 40 °C, 0 to 35 °C, 0 to 30 °C, 0 to 25 °C, 0 to 20 °C, 0 to 15 °C, 0 to 10 °C, 0 to 5 °C, 5 to 40 °C, 5 to 35 °C, 5 to 30 °C, 5 to 25 °C, 5 to 20 °C, 5 to 15 °C, 5 to 10 °C, 10 to 40 °C, 10 to 35 °C, 10 to 30 °C, 10 to 25°C, 10 to 20°C, 10 to 15°C, 15 to 40°C, 15 to 35°C, 15 to 30°C, 15 to 25°C, 15 to 20°C, 20 to 40°C, 20 to 35°C, 20 to 30°C, 20 to 25°C, 25 to 40°C, 25 to 35°C, or 25 to 30°C, but is not limited to.

The cooling step may utilize a conventional cooling method, such as using a cooler.

Arginine can be obtained through the concentration-crystallization process, and the liquid remaining after obtaining arginine can be separated as a mother liquor.

Step (b) may further comprise a step of drying the obtained arginine, and by removing the moisture, high-purity arginine can be finally obtained.

The arginine may be arginine crystals or arginine powder.

The conventional method for producing arginine included a decolorization process in the arginine production process, and thus had problems of treating waste activated carbon and wastewater generated in the decolorization process. In addition, the conventional method included the anion exchange process within the arginine production process, which resulted in a significant amount of time being required for arginine production.

However, in the method for producing arginine of the present disclosure, by omitting the decolorization process and separately performing the anion exchange process concurrently with the arginine production process, the yield of arginine can be improved and the time required for arginine production can be remarkably reduced.

Therefore, the method for producing arginine in the present application may be characterized in that the mother liquor separated in the concentration-crystallization step is seperated from the arginine production process, passed through an anion exchange resin in a separate process to remove impurities, and the resulting eluate is reused in step (a), step (b), or in both step (a) and step (b). These steps will be described in more detail in step (c) below.

### Step (c): A step of passing the mother liquor obtained in step (b) through an anion exchange resin (anion exchange process)

The method for producing arginine of the present application may comprise a step of passing the mother liquor separated in step (b) (concentration-crystallization step) through an anion exchange resin.

The anion exchange resin can remove anionic impurities. The anion exchange resin may be any commonly known anion exchange resin, such as a strongly basic anion exchange resin (SBA) or a weakly basic anion exchange resin (WBA), but is not limited thereto. The anion exchange resin may be a gel type or a porous type, but is not limited thereto.

The anionic impurities may include, but are not limited to, sulfate (SO₄²⁻), phosphate (PO₄²⁻), chloride ions (Cl⁻), and the like.

The mother liquor obtained by passing through the anion exchange resin may have the anionic impurities removed, thereby improving the color absorbance of the liquid or arginine as a final product.

Step (c) may be characterized by being spatially separated from steps (a) and (b).

That is, the anion exchange process of step (c) may be characterized by being spatially separated from, and concurrently performed with, the cation exchange process of step (a) and the concentration-crystallization process of step (b), which are the main processes for producing arginine.

Although the anion exchange process of step (c) is a time-consuming proc the overall time required for producing arginine can be dramatically reduced because it is performed concurrently with, and is spatially separated from, steps (a) and (b).

### Step (d): A step of reusing the eluate obtained in step (c)

Arginine is a highly soluble amino acid, and the mother liquor separated in step (b) also contains a large amount of arginine. Therefore, it is important to effectively reuse the mother liquor. Accordingly, the method for producing arginine of the present application may comprise a step of mixing the eluate obtained by subjecting the mother liquor to the anion exchange process with the arginine process liquid of step (a), step (b), or both steps (a) and (b), and repetitively performing the process.

Specifically, the arginine process liquid of step (a) may refer to (i) the fermentation broth containing arginine of step (a), (ii) an eluate obtained by subjecting the fermentation broth containing arginine to a cation exchange process, or (iii) a concentrate obtained by subjecting the eluate to a concentration process.

The arginine process liquid of step (b) may refer to a liquid to be treated with the concentration-crystallization process of step (b), i.e., an eluate obtained by subjecting the fermentation broth containing arginine to a cation exchange process in step (a), or a concentrate obtained by subjecting the eluate to a concentration process.

In this way, the mother liquor separated in step (b) is subjected to the anion exchange process as a separate process that is spatially separated from the arginine production process, and the eluate obtained by passing the anion exchange process is mixed with the arginine process liquid of step (a) and/or step (b) of the arginine production process, so that the anion exchange process and the arginine production process can be performed concurrently, thereby drastically reducing the time required for arginine production.

In the method for producing arginine of the present application, step (a) to step (d) may be performed repeatedly.

That is, the method for producing arginine the present application comprises:
(a) a step of passing a fermentation broth containing arginine through a cation exchange resin (cation exchange process);
(b) a step of concentrating and crystallizing the process liquid obtained in step (a) (concentration-crystallization process);
(c) a step of passing the mother liquor obtained in step (b) through an anion exchange resin (anion exchange process); and
(d): reusing the eluate obtained in step (c) in step (a), step (b), or both steps (a) step (b),
wherein the steps (a) through (d) may be performed repeatedly.

The method for producing arginine of the present application may be characterized in that a decolorization process is not performed.

The method for producing arginine of the present application exhibits no significant difference in the quality (purity, color absorbance, Bulk Density(BD)) of the final-produced arginine, although a decolorization process is not performed. Therefore, the method does not require the processes such as treatment of waste activated carbon and wastewater generated during the decolorization process, thereby achieving environmentally friendly and economical effects. Furthermore, by not performing a decolorization process, the time required for the decolorization process can be reduced.

The method for producing arginine of the present application performs the anion exchange process separately and concurrently with the arginine production process, thereby improving the arginine yield and significantly reducing the time required for arginine production compared with arginine production methods without separating anion exchange process.

In one example, the method for producing arginine of the present application can improve the arginine yield by 1% or more, 2% or more, or 3% or more, compared with a method for producing arginine that includes a decolorization process and/or does not differently separate anion exchange process, and due to this improvement in the arginine yield, the amount of raw materials and auxiliary materials used in arginine production can be reduced, thereby significantly reducing the manufacturing cost.

In one example, the method for producing arginine of the present application can reduce the time required for producing arginine by 1% or more, 2% or more, 3% or more, 4% or more, 5% or more, 6% or more, 7% or more, 8% or more, 9% or more, 10% or more, 15% or more, 20% or more, 25% or more, 30% or more, 35% or more, or 40% or more, compared with a method for producing arginine that includes a decolorization process and/or does not differently separate anion exchange process.

In one example, method for producing arginine of the present application can reduce the time required for producing arginine by about 1 hour or more, 2 hours or more, 3 hours or more, 4 hours or more, 5 hours or more, 6 hours or more, 7 hours or more, 8 hours or more, 9 hours or more, 10 hours or more, 15 hours or more, 20 hours or more, 25 hours or more, or 30 hours or more, compared with a method for producing arginine that includes a decolorization process and/or does not differently separate anion exchange process.

### [ADVANTAGEOUS EFFECTS]

The present application relates to a method for producing arginine in which a decolorization step is not performed and the anion exchange process is separated and performed concurrently with the arginine production process, thereby improving the yield of arginine and dramatically reducing the time required for production.

### [BRIEF DESCRIPTION OF THE DRAWING]

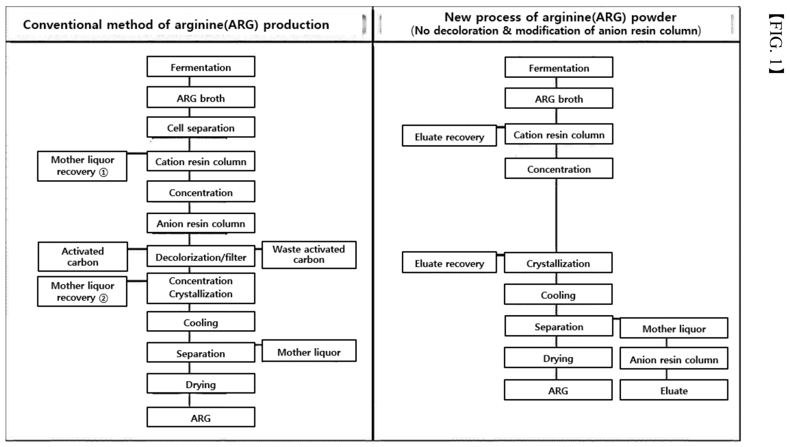

Fig. 1 shows a schematic diagram of a conventional arginine production method including a decolorization process and a method for producing arginine of the present application.

### [MODE FOR INVENTION]

The present invention will be described in more detail below with reference to the following examples. However, these examples are provided solely to illustrate the present invention, and the scope of the present invention is not limited by these examples.

The present application relates to a method for producing arginine in which a decolorization step is not performed and the anion exchange process is separated and performed concurrently with the arginine production process, thereby improving the yield of arginine and dramatically reducing the time required for production. In order to verify the effect of the present application, the method for producing arginine of the present application was compared with an conventional arginine production method that includes a decolorization process and performs the anion exchange process as part of the arginine production process, to show the results as below.

### Comparative Example 1. Conventional method for producing arginine

### a) Obtaining a fermentation broth containing arginine

To produce high-purity arginine using this process, arginine-producing microorganisms were cultured to obtain a fermentation broth containing arginine (arginine fermentation broth) having the composition shown in Table 1 below. The arginine fermentation broth contained the cultured microorganisms and medium components, and the moisture content and composition were analyzed to show them in Table 1 below.

**[Table 1]**

| Composition analysis of arginine fermentation broth | |
|---|---|
| Composition | Numerical Value |
| Arginine | 130.6 g/L |
| Amino acids other than arginine | 2.6 g/L |
| Inorganic substances | 62.0 g/L |
| Organic acid | 0.3 g/L |
| water | 79.2% |

The pH of the arginine fermentation solution was adjusted downward to 4.0 by treating with sulfuric acid, and the microbial cells used for arginine fermentation were separated or filtered using a cell separation facility (e.g., Membrane filter, Mechanical Separator, etc.).

### b) Step of passing the arginine fermentation solution through a cation exchange resin (cation exchange process)

The arginine fermentation broth, from which the microbial cells had been removed, was passed through a cation exchange resin (MC-08, Samyang Corporation) to obtain the arginine eluate, in which anions were separated through resin-arginine adsorption/elution. The anion separation efficiency in the cation exchange process was approximately 99%. Ammonia (Daejung Chemicals & Metals) was used as the eluent to desorb the arginine adsorbed to the resin in the cation exchange process.

### c) Step of removing ammonia and concentrating arginine

The arginine eluate (liquid passed through the ion exchange resin) obtained in step b) was concentrated using a post-vacuum Rotary Evaporator (BUCHI, Rotavapor R-300) at an internal temperature of 55°C, so that it degassed ammonia and increased from approximately 72 g/L to 200 g/L of the arginine concentration.

### d) Step of passing the concentrate obtained in step c) through an anion exchange resin (anion exchange process)

The entire concentrate obtained in step c) was passed through an anion exchange resin (AMP-26, Samyang Corporation) to remove co-existing byproducts with absorption, to recover the arginine process liquid.

### e) Step of activated carbon treatment (decolorization)

To improve the light transmittance of the process liquid passing through the anion exchange resin, the temperature of the process liquid was raised to 60°, and was stirred for approximately 1 hour with addition of activated carbon at a ratio of 3% by weight to arginine. The activated carbon was then removed using a Nutsche filter and filter paper, to produce a filtrate.

### f) Step of performing a concentration-crystallization process on the filtrate obtained in step e) to obtain arginine crystals (concentration crystallization process).

The filtrate was a process liquid of high-purity arginine. When the concentration-crystallization process was performed with a vacuum rotary evaporator at an internal temperature of 50°C to concentrate the arginine filtrate from 200 g/L to approximately 500-550 g/L, arginine in excess of its solubility precipitated as crystals. After the concentration-crystallization process was complete, the concentrate was cooled to 25-30°C using a cooling circulator to increase the yield of arginine crystals by decreasing solubility through lowering the temperature. The precipitated arginine crystals and the mother liquor were separated using a solid-liquid separator (e.g., a basket separator). The separated arginine crystals were dried in an oven dryer to remove moisture, to finally obtain high-purity arginine.

### g) Step of reusing the mother liquor obtained in step e)

Since arginine is an amino acid with relatively high solubility, a large amount of arginine remains in the mother liquor generated during the separation of arginine crystals (i.e., the liquid remaining after separating arginine crystals by performing a concentration-crystallization process on a process liquid containing arginine). Therefore, in order to increase the process recovery rate, the mother liquor was returned to the arginine eluate of step (b) and/or the arginine process liquid of step (f), and the process was repeatedly performed.

The physiochemical properties of each process liquid of the conventional arginine production method in Comparative Example 1 are summarized in Table 2 below.

**[Table 2]**

| Comparison of physiochemical properties by each process liquid in conventional arginine production method | | | | | | | |
|---|---|---|---|---|---|---|---|
| Item | Unit | Process liquid | | | | | |
| | | Fermentatio n broth | Cell separation | Cation exchange process | Concentrati on | Anion exchange process | decolorizati on /filteration |
| Experimental Equipment/mat erial | | - | Membrane Filter | Cation resin (MC-08) | Rotary Evaporator | Anion resin (AMP-26) | Activated carbon Nutsche filter |
| Residen ce time | hr | 4 | 5 | 10 | 12 | 20 | 6 |
| pH | - | 7.0 | 4.0 | 11.6 | 10.7 | 10.7 | 11.1 |
| Temper ature | °C | 30 | 60 | 45 | 55 | 40 | 60 |
| Concent ration | g/L | 130 | 130 | 72 | 200 | 205 | 204 |
| Purity | % | 56.1 | 60.0 | 93.1 | 93.4 | 96.1 | 96.6 |
| *color absorba nce | abs | 141.6 | 12.1 | 0.43 | 1.17 | 0.03 | 0.02 |

| | | | | | | | |
|---|---|---|---|---|---|---|---|
| *Color absorbance: The color value at 430 nm (lower values indicate a clearer solution). *Purity: (arginine weight / total solids weight in the process liquid) × 100 | | | | | | | |

As shown in Table 2, the anion exchange process is the step that requires the most time among all processes.

### Example 1. Method for producing arginine without decolorization process

In the conventional arginine production method described in Comparative Example 1, (1) the decolorization process (step e)) was not performed, and (2) the anion exchange process (step d)) was separated as an independent process and performed concurrently with the arginine production process by treating the mother liquor generated in step (f).

That is, steps a) to c) of Comparative Example 1 were performed in the same manner, but the arginine concentrate obtained in step c) was subjected directly to the concentration-crystallization process of step f), to separate the arginine crystals and mother liquor.

Since the anion exchange process is a time-consuming step, in the present application, the anion exchange process was separated as an independent process, and the eluate obtained by performing the anion exchange process on the mother liquor separated in the concentration-crystallization step (i.e., the mother liquor passed through the anion exchange resin) was mixed with the arginine eluate of step (b) and/or the concentrate fed into the concentration-crystallization process of step (f), and the process was repeatedly performed.

The qualities of the mother liquor obtained in the arginine production process and the eluate obtained by passing the mother liquor through an anion exchange resin are summarized in Table 3 below.

**[Table 3]**

| Process liquid | Item | Unit | Anion exchange process and decolorization for mother liquor | | | |
|---|---|---|---|---|---|---|
| | | | average | 1 cycle | 2 cycle | 3 cycle |
| Mother liquor | Purity | % | 87.9 | 87.4 | 88.0 | 88.4 |
| | Color absorban ce | abs | 1.75 | 1.64 | 1.84 | 1.76 |
| Eluate | Purity | % | 92.4 | 93.0 | 92.8 | 91.5 |
| | Color absorban ce | abs | 0.11 | 0.17 | 0.07 | 0.09 |
| Purity difference | | % | 4.5 | 5.7 | 4.8 | 3.2 |
| Decolorization efficiency | | % | 93.7 | 89.7 | 96.2 | 95.2 |

| | | | | | | |
|---|---|---|---|---|---|---|
| *Color absorbance: The color value at 430 nm (lower values indicate a clearer solution). | | | | | | |

As shown in Table 3 above, the eluate that passed through the anion exchange resin had improved purity and color absorbance compared to the mother liquor.

The eluate passed through the anion exchange resin was mixed with the arginine eluate of step (b) (cation exchange process) and the concentrate to be input into step (f) (concentration-crystallization process), and passed through the process repetitively.

### 3. Comparison of results

When comparing the quality of arginine produced by reusing the eluate obtained by passing the mother liquor separated during the arginine production process through an anion exchange resin without performing the decolorization process as in Example 1, with the quality of arginine produced using the conventional production method (Comparative Example 1), there was no significant difference in main qualities (purity, pH, BD, etc.) (Table 4 below).

**[Table 4]**

| Comparison of main qualities and process indicators | | | | |
|---|---|---|---|---|
| Item | Detailed item | Unit | Comparative Example 1 | Example 1 |
| Product quality | purity | % | 99.3 | 98.7 |
| | pH | - | 10.8 | 10.7 |
| | Bulk Density | g/ml | 700±20 | 680±30 |
| Process treatment time | hour | 72 | 46 | |
| Process yield | % | 88.5 | 90.5 | |

However, the method for producing arginine of the present application reduced arginine degradation losses that occurred during the decolorization process by not performing the decolorization process, thereby increasing the yield of the production process by about 2% and reducing the time required for the decolorization process, and decreased the time required for the anion exchange process by performing the anion exchange process separately from and concurrently with the arginine production process, thereby achieving the effect of improving productivity through a time reduction of about 36% of the entire process.

In addition, by not performing the decolorization process, the cost required for the decolorization process can be reduced, an environmentally friendly effect can be achieved by eliminating the need of disposal of waste activated carbon, and the yield of arginine is improved by reducing the amount of raw materials and auxiliary materials, which can significantly reduce the manufacturing cost.

From the above description, those skilled in the art will understand that the present invention can be implemented in other specific forms without altering its technical spirit or essential characteristics. In this regard, it should be understood that the embodiments described above are illustrative in all respects and not restrictive. The scope of the present invention should be interpreted as encompassing all changes or modifications derived from the meaning and scope of the following claims and their equivalent concepts, rather than the detailed description above.

## Claims

1. A method for producing arginine comprising:
(a) passing a fermentation broth containing arginine through a cation exchange resin to obtain an eluate;
(b) concentrating and crystallizing the eluate obtained in step (a);
(c) passing the mother liquor obtained in step (b) through an anion exchange resin to obtain an eluate; and
(d) reusing the eluate obtained in step (c) in step (a), (b), or both steps (a) and (b),
wherein steps (a) through (d) are repetitively performed.

2. The method according to Claim 1, wherein the fermentation broth containing arginine is a fermentation product obtained by culturing a microorganism producing arginine, or a fermentation product from which the cells are removed.

3. The method according to Claim 1, wherein step (a) further comprises a step of concentrating the eluate passed through the cation exchange resin to a concentration of 200 g/l or less,

4. The method according to Claim 1, wherein step (b) is to concentrate arginine to a concentration of 200 g/l or more of arginine concentration.

5. The method according to Claim 1, wherein the concentrating in step (b) is performed at 30 to 60 °C.

6. The method according to Claim 1, wherein step (b) further comprises a step of cooling the concentrate to 35 °C or less.

7. The method according to Claim 1, wherein step (b) further comprises a step of drying the obtained arginine.

8. The method according to Claim 1, **characterized in that** step (c) is performed concurrently with steps (a) and (b).

9. The method according to any one of Claim 1 to Claim 8, **characterized in that** a decolorization process is not performed'

10. The method according to any one of Claims 1 to 8, wherein the time required for producing arginine is reduced by 20% or more, compared with a method for producing arginine in which the anion exchange process is not performed separately.

11. The method according to any one of Claims 1 to 8, wherein the method for producing arginine is a method for producing arginine crystals or arginine powder.
